# EUROPEAN PATENT SPECIFICATION

(11) **EP 2 356 246 B1**
(45) Date of publication and mention of the grant of the patent: **22.08.2012**
(21) Application number: 08875859.4
(22) Date of filing: 17.11.2008
(51) Int. Cl.: C12P 19/38, C12N 9/10

(54) **Biocatalytic preparation of 5-methyluridine**
Biokatalytische Herstellung von 5-Methyluridin
Préparation biocatalytique de 5-méthyle uridine

(30) Priority: 14.11.2008 WO PCT/IB2008/054778
(43) Date of publication of application: 17.08.2011
(73) Proprietor: CSIR, 0002 Pretoria (ZA)
(72) Inventor: VISSER, Daniel, Finsch, 1448 Alberton (ZA); GORDON, Gregory, Ernest, Robert, 2195 Johannesburg (ZA); HENNESSY, Fritha, 2195 Johannesburg (ZA); RASHAMUSE, Konanani, Justice, 2090 Johannesburg (ZA); BRADY, Dean, 1684 Johannesburg (ZA); BODE, Moira, Leanne, 1684 Johannesburg (ZA); LOUW, Maureen, Elizabeth, 0181 Waterkloof (ZA)
(74) Representative: Murray, Adrian D'Coligny
(86) International application number: PCT/IB2008/054810
(87) International publication number: WO 2010/055369

(56) References cited:
- TAKAMI ET AL: "Complete genome sequence of the alkaliphilic bacterium Bacillus halodurans and genomic sequence comparison with Bacillus subtilis" NUCLEIC ACIDS RESEARCH, vol. 28, 2000, pages 4317-4331, XP008044903
- DATABASE EMBL/EBI 1 October 2000 (2000-10-01), TAKAMI ET AL: "Complete genome sequence of the alkaliphilic bacterium Bacillus halodurans and genomic sequence comparison with Bacillus subtilis" XP002535102 Database accession no. Q9KCN8
- DATABASE EMBL/EBI 27 April 1998 (1998-04-27), TOSHITADA ET AL: "Purine nucleoside phosphorylase" XP002535103 Database accession no. E61303
- ISHII ET AL: "Enzymatic production of 5-methyluridine from purine nucleosides and thymine by Erwinia carotovora AJ-2992" AGRICULTURAL AND BIOLOGICAL CHEMISTRY, vol. 53, 1989, pages 3209-3218, XP002535185
- ROCCHIETTI ET AL: "Immobilization and stabilization of recombinant multimeric uridine and purine nucleoside phosphorylases from Bacillus subtilis" BIOMACROMOLECULES, vol. 5, 2004, pages 2195-2200, XP002535186
- URBONAVICIUS ET AL: "Identification of a novel gene encoding a flavin-dependent tRNA:m(5)U methyltransferase in bacteria - evolutionary implications" NUCLEIC ACIDS RESEARCH, vol. 33, 2005, pages 3955-3964, XP002535187
- GORDON ET AL: "Biocatalytic preparation of 5-methyluridine (5-MU)" November 2008 (2008-11), page 1, XP002535188 Retrieved from the Internet: URL:researchspace.csir.co.za/dspace/bitstr eam/10204/2687/1/Gordon_P_2008.pdf> [retrieved on 2009-07-02]

## Description

THIS INVENTION relates to nucleosides. It relates in particular to a process for manipulating a nucleoside, and to a biocatalyst for use in the process.

Modified nucleosides and nucleotides can be used as antiviral therapeutic agents, or as precursors for such agents. An example of an important nucleoside of this kind is ß-thymidine. ß-thymidine can be obtained from 5-methyluridine which is, however, a relatively expensive intermediate so that the cost of producing the antiretrovirals AZT and stavudine therefrom is correspondingly high. It is thus an object of this invention to provide a means whereby 5-methyluridine can be produced more cost effectively.

The enzyme BH1531 is known from Takami et al, "Complete genome sequence of the alkaliphilic bacterium Bacillus halodurans and genomic sequence comparison with Bacillus subtilis", Nucleic Acids Research, vol 28, 2000, pages 4317-4331, as well as from Database EMBL/EBI, 1 October 2000 (2000-10-01), Takami et al, "Complete genome sequence of the alkaliphilic bacterium Bacillus halodurans and genomic sequence comparison with Bacillus subtilis", XP002535102 Database accession no Q9KCN8; however, its use as a biocatalyst, in combination with another enzyme, for converting guanosine to 5-methyluridine is not known.

According to a first aspect of the invention, there is provided a method for converting guanosine to 5-methyluridine by means of biocatalytic transglycosylation, with the biocatalyst comprising a combination of a purine nucleoside phosphorylase from *Bacillus halodurans,* PNPase (BH1531) of sequence ID No 1, or a protein with at least 90% sequence similarity thereto, and a pyrimidine nucleoside phosphorylase (PyNPase).

In one embodiment of the invention, the pyrimidine nucleoside phosphorylase may be from *Escherichia coli,* particularly *E. coli* UPase of sequence ID No 2, or a protein with at least 90% sequence similarity thereto.

In another embodiment of the invention, the pyrimidine nucleoside phosphorylase may be from *Bacillus halodurans,* particularly *B.halodurans* PyNPase (BH1533) of sequence ID No 3, or a protein with at least 90% sequence similarity thereto.

The conversion thus involves the transfer of ribose-1-phosphate from the guanosine to a first nitrogenous base, thereby to obtain the 5-methyluridine and a second nitrogenous base. In general, the biocatalytic transglycosylation reaction occurs by the transfer of a ribose-1-phosphate from a nucleoside to a purine or pyrimidine base in the presence of phosphate ions. The first nitrogenous base may typically be thymine, while the second nitrogenous base may typically be guanine.

The biocatalyst is thus a combination of a purine nucleoside phosphorylase (PNPase), *B. halodurans* PNPase (BH1531), of sequence ID No 1 (associated nucleotide sequence given as sequence ID No. 4), or a protein with at least 90% sequence similarity thereto, and a pyrimidine nucleoside phosphorylase (PyNPase), *E. coli* uridine phosphorylase (UPase), of sequence ID No 2 (associated nucleotide sequence given as sequence ID No. 5), or *B. halodurans* BH1533 of sequence ID 3 (associated nucleotide sequence given as sequence ID No. 6) or a protein with at least 90% sequence similarity to either of these.

The enzymes may be isolated and over-expressed in an *E. coli* protein expression system.

The *B. halodurans* may be a strain of gram positive *B. halodurans* Alk36 bacteria deposited under accession number NCIMB41348 on 23 November 2005 at NCIMB Ltd of Ferguson Building, Craibstone Estate, Buchsburn, Aberdeen AB21 9YA. Due to the high sequence identity between the genomes of *B. halodurans* Alk36 and *B. halodurans* C-125, primers for the cloning of the purine and pyrimidine nucleoside phosphorylase genes were designed according to the sequenced genome of *B. halodurans* C-125 which is published in the DNA Data Bank of Japan (http://www.ddbj.nig.ac.jp). The genes annotated BH1531 and BH1533, according to GenBank, encode a purine nucleoside phosphorylase and a pyrimidine nucleoside phosphorylase respectively.

The purine nucleoside phosphorylase and pyrimidine nucleoside phosphorylase may be heterologously expressed in a suitable host organism such as *E. coli.* For example, expression of PNPase and UPase in *E. coli* BL21 (DE3) [pMS1531] and *E. coli* BL21 (DE3) [pETUP] respectively can be performed. Production may be achieved by culturing the host organism by fermentation either in batch, fed batch or in continuous mode.

Generally, the biocatalyst may be the purified enzymes, active cell lysates, cell pastes, whole cells or immobilised forms of each of these.

In one embodiment, the biocatalyst may be in the form of a free suspension. However, in another embodiment, the biocatalyst may be immobilized on a backbone. Thus, the biocatalyst can be in the form of an enzyme structure or particle, such as that described in WO 2005/080561, or in the form of an emulsion-derived particle as described in ZA 2007/09300 and in which the enzymes are bonded to lattices of the particles. This will permit the biocatalyst to be recovered and recycled after completion of the transglycosylation.

The transglycosylation may be performed at between 30°C and 70°C, preferably at between 40°C and 70°C, typically at 55°C to 65°C.

The transglycosylation may be effected over a pH range of 6 to 11, preferably at a pH of between 7 to 8.5. The reaction may be carried out using phosphate buffer, as a reaction medium. The phosphate buffer may be at a concentration of 20 - 250 mM, but preferably 25 - 50 mM.

The initial concentration of the guanosine in the reaction medium, in which the transglycosylation is carried out, may be from 1 to 19.5% m/m, preferably from 8 to 13% m/m.

The initial concentration of the first nitrogenous base in the reaction medium may be from 1 to 15% m/m, preferably from 4 to 6% m/m.

The transglycosylation reaction may be carried out using a first nitrogenous base (thymine) to first nucleoside (guanosine) ratio from 2.3:1 to 1:1, preferably from 1:1 to 1.15:1.

The molar yield of 5-methyluridine may be between 30 - 90%, and typically 80% - 90%.

The transglycosylation reaction productivity may be between 0.5 and 27 g/l/h, but typically at 7 - 11 g/l/h.

The method may include recovering the 5-methyluridine. In particular, the method may include recovering or separating the 5-methyluridine from the second nitrogenous base (guanine) using solubility differences. Thus, when the second nitrogenous base is guanine, the 5-methyluridine is largely soluble and the guanine largely insoluble in the reaction medium at the temperatures described above. This facilitates separation thereof. Using this method, recoveries of 80 - 90% from the transglycosylation medium are achievable. Together with the 5-methyluridine reaction yields of 80 - 90%, this gives isolated yields of 5-methyluridine of between 64 - 80% from the biocatalytic reaction.

For the biocatalytic reaction, ie the transglycosylation reaction, a phosphate buffer may first be introduced into a suitable reaction vessel or container, whereafter guanosine, preferably in solid particulate form, and additional phosphate buffer, may be added to the reaction vessel. Thymine may be pre-wetted using phosphate buffer, before being transferred to the reaction vessel. The various constituents introduced into the reaction vessel constitute a reaction medium for the transglycosylation reaction. These substances, in phosphate buffer, may be heated in the vessel to the reaction temperature as hereinbefore set out, and maintained at the reaction temperature for a period of time, typically about 2 hours, before addition of the biocatalyst to the reaction vessel. The biocatalyst components (PNPase and PyNPase/UPase) may be pre-dissolved in phosphate buffer prior to addition thereof to the reaction vessel.

The process may include chemically manipulating the 5-methyluridine to obtain therefrom ß-thymidine. The chemical manipulation of the 5-methyluridine may be effected without any purification thereof after it has been obtained by the conversion of the guanosine.

The chemical manipulation of the 5-methyluridine may include reacting the 5-methyluridine with hydrogen bromide or acetyl bromide in acetonitrile, as the first step towards obtaining ß-thymidine (C₁₀H₁₄N₂O₅).

The process of the invention is thus a process whereby ß-thymidine can be produced synthetically. The final yield of the ß-thymidine over the chemical manipulation steps may be 65 to 80%, typically 70 - 75%.

According to a second aspect of the invention, there is provided a biocatalyst which comprises the combination of a pyrimidine nucleoside phosphorylase (PyNPase), and a purine nucleoside phosphorylase *B. halodurans,* PNPase (BH1531) of sequence ID No 1, or a protein with at least 90% sequence similarity thereto.

As indicated hereinbefore, the pyrimidine nucleoside phosphorylase may, in one embodiment of the invention, be from *Escherichia coli,* particularly *E. coli* UPase of sequence ID No 2, or a protein with at least 90% sequence similarity thereto.

In another embodiment of the invention, the pyrimidine nucleoside phosphorylase may be from *Bacillus halodurans,* particularly *B.halodurans* PyNPase (BH1533) of sequence ID No 3, or a protein with at least 90% sequence similarity thereto.

The invention will now be described in more detail, with reference to the accompanying non-limiting examples and the accompanying drawings and sequence listings.

In the drawings
FIGURE 1 shows the results obtained in Example 3, i.e. comparative efficiencies of combinations of purine and pyrimidine nucleoside phosphorylases in the production of 5-methyluridine (5-MU). Lane 1: PNP:rUP; Lane 2: PNP:BH1533; Lane 3: PNP:KP PyNP; Lane 4: PNP:BL PyNP; Lane 5: XapA:rUP; Lane 6: XapA:BH1533; Lane 7: XapA:BL PyNP; Lane 8: XapA:KP PyNP; Lane 9: BH1531:rUP; Lane 10: BH1531:BH1533; Lane 11: BH1531:BL PyNP; Lane 12: BH1531:KP PyNP.
FIGURE 2 shows (Example 4) overexpression of *E. coli* uridine phosphorylase in *E. coli* BL21, and in which Lane 1: Marker; Lane 2: *E. coli* BL21 [pET20b] (negative control); Lane 3 to 8: *E. coli* BL21 [pETUP] (various constructs).
FIGURE 3 shows (Example 4) over-expression of *B. halodurans* BH1531 in *E. coli* BL21, and in which Lane 1: marker; Lane 2: *E. coli* BL21; Lane 3: *E. coli* BL21 [pMS470]; Lane 4: *E. coli* BL21 [pMS1531] (uninduced); Lanes 5 to 10: *E. coli* BL21 [pMS1531] (various constructs) (induced).
FIGURE 4 shows the effect of pH on the guanosine conversion and 5-methyluridine yield as described in Example 6. Clear bars show guanosine conversion (Conv.). Shaded bars show 5-methyluridine yield. Standard deviations represent the average of three experiments.
FIGURE 5 shows the effect of temperature on the guanosine conversion and 5-methyluridine yield as described in Example 7. The symbol ◆ represents guanosine conversion at 40°C; Δ represents 5-methyluridine yield at 40°C; ▲ represents guanosine conversion at 50°C; ■ represents 5-methyluridine yield at 50°C; * represents guanosine conversion at 60°C; • represents 5-methyluridine yield at 60°C; ○ represents guanosine conversion at 70°C; □ represents5-methyluridine yield at 70°C.
FIGURE 6 shows the yield and productivity of the biocatalytic reaction at increased substrate concentrations as described in Example 8. The symbol ▲ represents 5-methyluridine reactor productivity; ◆ represents guanosine conversion; ■ represents 5-methyluridine yield.
FIGURE 7 shows the transglycosylation percent recovery and productivity obtained over time at 1 I scale, see Example 9. The symbol ▲ represents 5-methyluridine reactor productivity; ◆ represents guanosine conversion; ■ represents 5-methyluridine yield.
FIGURE 8 shows the guanosine conversion rate with reaction time over three replicate experiments at 20 I scale as described in Example 9. The symbol A represents guanosine conversion 1; ◆ represents guanosine conversion 2; ■ represents guanosine conversion 3.
FIGURE 9 shows the 5-methyluridine yield with reaction time over three replicate experiments at 20 I scale as described in Example 9. The symbol A represents 5-methyluridine yield 1; ◆ represents 5-methyluridine yield 2; ■ represents 5-methyluridine yield 3.
FIGURE 10 shows the reactor productivity of 5-methyluridine with reaction time over three replicate experiments at 20 I scale as described in Example 9. The symbol ▲ represents 5-methyluridine reactor productivity 1; ◆ represents 5-methyluridine reactor productivity 2; ■ represents 5-methyluridine reactor productivity 3.

### Sequence listings

- Sequence ID No 1:: Amino acid sequence of *B. halodurans* BH1531
- Sequence ID No. 2:: Amino acid sequence of *E. coli* UPase
- Sequence ID No. 3:: Amino acid sequence of *B. halodurans* BH1533
- Sequence ID No. 4:: Nucleotide sequence of the *B. halodurans* BH1531 gene which encodes the protein sequence of sequence ID No. 1
- Sequence ID No. 5:: Nucleotide sequence of the *E. coli* UPase gene which encodes the *E. coli* UPase enzyme of protein sequence ID No. 2
- Sequence ID No. 6:: Nucleotide sequence of the *B. halodurans* BH1533 gene which encodes the protein sequence of sequence ID No. 3

In the Examples hereunder, for the biocatalytic reactions, phosphate buffer is added first to a suitable reactor, followed by solid particulate guanosine and additional buffer. Thymine is pre-wetted using phosphate buffer prior to transfer to the reaction vessel. These substrates, in phosphate buffer, are heated in the reactor at the final reaction temperature for 2 h prior to addition of the biocatalysts. The biocatalysts (PNPase and PyNPase) are pre-dissolved in phosphate buffer prior to their addition to the reaction vessel.

### EXAMPLE 1

Using commercial enzymes, thymidine (a stavudine and AZT precursor) was produced (with deoxyguanosine and deoxyinosine as donors), and the reaction equilibrium could be modified by the inclusion of xanthine oxidase.

PNPase (from *Cellumonas sp.),* thymidine phosphorylase (TPase) and xanthine oxidase (XO) were purchased from Sigma-Aldrich (Pty) Ltd. The nucleosides were also purchased from Sigma-Aldrich (Pty) Ltd.

Three ml reactions were set up in 50 mM phosphate buffer, pH 7.4. Varying nucleosides and enzymes were incubated at 25°C for one to three hours, with shaking. Samples were taken at set time points and analysed using HPLC. All nucleoside stock solutions were made to 50 mM concentration, with a final concentration in the reaction of 2.5 mM unless otherwise stated.

Analysis by HPLC was performed using a Chromolith speed rod 18e Column, and an eluent of 5% methanol, 95% of a 0.1% sodium phosphate buffer, pH 7.5. The flow rate used was 0.5 ml/min. Nucleosides and bases were detected by diode array spectrophotometry at 260 nm. Results are given in Table 1.

**Table 1: Summary of results obtained for reactions**

| Rxn | Starting reagents | Enzymes | Expected product¹ | (% total peak area) |
|---|---|---|---|---|
| 1 | thymidine | TPase | thymine | 78.5 |
| 2 | thymine, 2'-deoxyribose-1-phosphate | TPase | thymidine | 19.5 |
| 3 | inosine | XO, PNPase | hypoxanthine plus Xanthine | 61.7 |
| 4 | hypoxanthine, 2'-deoxyribose-1-phosphate | XO, PNPase | 2'-deoxyinosine | 33.4 |
| 5 | 50 mM guanosine, thymine | TPase, PNPase | 5-methyluridine | 0 |
| 6 | 200 mM guanosine, thymine | TPase, PNPase | 5-methyluridine | 0 |
| 7 | 100 mM thymine, guanosine | TPase, PNPase | 5-methyluridine | 0 |
| 8 | 150 mM thymine, guanosine | TPase, PNPase | 5-methyluridine | 0 |
| 9 | 250 mM thymine, guanosine | TPase, PNPase | 5-methyluridine | 0 |
| 10 | inosine, thymine | XO, TPase, PNPase | 5-methyluridine | 0 |
| 11 | 5-methyluridine | TPase | thymine | 19.6 |
| 12 | 2'-deoxyinosine; 50 mM phosphate, thymine | XO, TPase, PNPase | thymidine | 2.7 |
| 13 | 2'-deoxyinosine; 25 mM phosphate, thymine | XO, TPase, PNPase | thymidine | 3.7 |
| 14 | 2'-deoxyinosine; 5 mM phosphate, thymine | XO, TPase, PNPase | thymidine | 7.2 |
| 15 | 2'-deoxyinosine; Tris, thymine | XO, TPase, PNPase | thymidine | 10 |

| | | | | |
|---|---|---|---|---|
| ¹ - The product of interest is indicated here. | | | | |

Reactions 1 and 2 confirmed that the forward and reverse pyrimidine reactions can occur when using TPase. Reactions 3 and 4 confirmed that forward and reverse purine reactions can occur when using XO and PNPase.

The aim of the inventors was to transfer the ribose group from guanosine to thymine (i.e. from a purine to a pyrimidine), yielding 5-methyluridine (Reactions 5 and 6). These reactions were unsuccessful as no 5-methyluridine was produced. Reactions 5 to 9 show that the reaction was not easily achieved using commercially available enzymes, as no 5-methyluridine was produced.

An alternative purine was tried, with thymine and inosine as reagents to generate 5-methyluridine, with inosine providing ribose-1-phosphate, and hypoxanthine being converted to uric acid to prevent the reverse reaction occurring (Reaction 10). The reverse reaction of 5-methyluridine to thymine and ribose-1-phosphate was successfully performed (Reaction 11).

Altering the buffer conditions used in the formation of thymidine using 2'-deoxyinosine allowed for an increase in the relative proportions of thymidine. Decreasing the amount of phosphate present, or using a Tris buffer in the reaction allowed for a shift in the equilibrium towards the formation of thymidine from thymine and 2'-deoxyribose-1-phosphate, as opposed to the breakdown of thymidine to thymine with the release of phosphate (Reactions 12 to 15).

Hence, with the combined PNPase and TPase commercial enzymes, it was not possible to perform transglycosylation with ribose-1-phosphate. On this basis, the Inventors decided to isolate nucleoside phosphorylases from other sources.

### EXAMPLE 2

Biomass of *E. coli* and *B. halodurans* was obtained by growth of each of the organisms at 37°C in LB broth. The biomass was disrupted through sonication and the cytosolic fraction separated from the cellular debris by centrifugation (13 000 x g). This cytosolic fraction was used as the crude extract of each of the organisms.

Three ml reactions were set up in 50 mM phosphate buffer, pH 7.4. Varying nucleosides, enzymes and crude extract preparations were incubated at 25°C for one to sixteen hours, with shaking. Samples were taken at set time points and analysed using HPLC. All nucleoside stock solutions were made to 50 mM concentration, with a final concentration in the reaction of 2.5 mM.

Analysis by HPLC was performed using a Chromolith speed rod 18e Column, and an eluent of 5 % methanol and 95 % of a 0.1 % sodium phosphate buffer, pH 7.5. The flow rate used was 0.5 ml/min. Nucleosides and bases were detected by diode array spectrophotometry at 260 nm.

Through use of the partially purified *E. coli* or *B. halodurans* cell extracts (which contained intrinsic PNPase and PyNPase activities), it was possible to generate both thymidine and 5-methyluridine. Both *E. coli* and *B. halodurans* crude extracts showed production of 5-methyluridine using either inosine or guanosine as the ribose-1-phosphate donor. The yields of the reactions are summarised in Table 2.

**Table 2: Transglycosylations of nucleosides using nucleosidases**

| Rxn | Starting nucleoside | Biocatalyst | Product | Max Yield (%) |
|---|---|---|---|---|
| 1 | Inosine, thymine | Crude extract (*E. coli)*, XO | 5-methyluridine | 38 |
| 2 | Inosine, thymine | Crude extract (*B. halodurans*), XO | 5-methyluridine | 21.8 |
| 3 | 2'-deoxyinosine, thymine | Crude extract (*E. coli*), XO | Thymidine | 24 |
| 4 | 2'-deoxyinosine, thymine | Crude extract (*B. halodurans),* XO | Thymidine | 20.5 |
| 5 | Guanosine, thymine | Crude extract (*E. coli*) | 5-methyluridine | 30 |
| 6 | Guanosine, thymine | Crude extract (*B. halodurans*) | 5-methyluridine | 60.2 |
| 7 | 2'-deoxyguanosine, thymine | Crude extract (*E. coli*) | Thymidine | 49 |
| 8 | 2'-deoxyguanosine, thymine | Crude extract (*B. halodurans*) | Thymidine | 41.0 |

### EXAMPLE 3

A number of enzymes and combinations thereof were tested for the ability to produce 5-methyluridine by transglycosylation. The enzymes tested were:
Purine Nucleoside Phosphorylases (PNPases)
   - Recombinant *E. coli* PNP (PNP)
   - Recombinant *E. coli* Xanthosine phosphorylase (XapA)
   - Recombinant *B. halodurans* PNP (BH1531)
Pyrimidine Nucleoside Phosphorylase (PyNPases)
   - Recombinant *E. coli* Uridine phosphorylase (rUP)
   - Native *B. halodurans* PyNPase (BH1533)
   - Native *Klebsiella pneumoniae* PyNPase (KP PyNP)
   - Native *Bacillus licheniformis* PyNPase (BL PyNP)

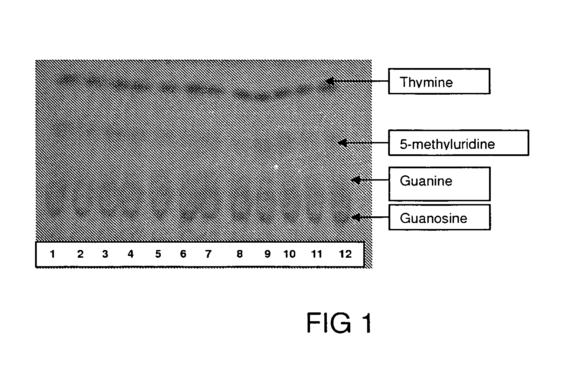

The total enzyme concentration for each reaction was maintained at 0.004 U/ml. Enzyme solutions and assay reagent (100 µl containing 5 mM guanosine and 5 mM thymine in 50 mM pH 8.0 phosphate buffer) were aliquoted into a 96-well microtitre plate. The microtitre plate was incubated for 1 h at 40°C with shaking at 900 rpm. Reactions were analysed by TLC (5 µl spot, 85:15 chloroform:methanol mobile phase, 10 cm UV₂₅₄ Silica plates). The results are given in Figure 1.

In Figure 1, when comparing combinations of purine and pyrimidine nucleoside phosphorylases, it is evident that the combinations of PNP:rUP (lane 1) and BH1531:rUP (lane 9) gave the highest 5-methyluridine synthesis at 40°C. XapA showed little to no 5-methyluridine production. Similarly, the PyNPases from *E. coli* (rUP) and *B. halodurans* (BH1533) seemed far superior catalysts when compared to those from *K. pneumoniae* and *B. licheniformis.*

### EXAMPLE 4

### Expression and purification of Nucleoside Phosphorylases from E. coli and B. halodurans

### Genomic DNA isolation

*E. coli* XL1 blue was grown overnight in a 10 ml culture volume. An aliquot of 1.5 ml of this was pelleted, and genomic DNA was isolated using the Qiagen DNeasy plant mini kit (Qiagen) using the standard protocol. *B. halodurans* genomic DNA was isolated similarly. The *B. halodurans* was a strain of gram positive *B. halodurans* Alk36 bacteria deposited under accession number NCIMB43148.

### PCR Amplification

Oligonucleotides to amplify the UPase, PNPase, XapA and TPase genes from *E. coli* were designed based on published sequences (Esipov *et al,* 2002). Due to the high sequence identity between the genomes of *B. halodurans* Alk36 and *B. halodurans* C-125, primers for the cloning of the purine and pyrimidine nucleoside phosphorylase genes were designed according to the sequenced genome of *B. halodurans* C-125 (GenBank accession number BA000004). The genes for the purine nucleoside phosphorylases were annotated as PH1531 and BH1532 and the gene for the pyrimidine nucleoside phosphorylase was annotated as BH1533. PCR was performed using genomic DNA as the template, and using the High Fidelity amplification kit (Roche). Again standard protocols were followed. The PCR cycling parameters were as follows: one hold at 95°C for 5 minutes, followed by 30 cycles of 95°C for 1 minute, 50°C for 1 minute and 72°C for 1 minute. The resultant product was held at 4°C until analysis on a 0.8% agarose gel was performed.

PCR successfully amplified the coding regions of BH1531 and *E. coli* UPase (Sequence ID No. 4, sequence ID No. 5, respectively) with the associated amino acid sequences given in sequence ID No. 1 and sequence ID No. 2 respectively.

### Cloning of nucleoside phosphorylase genes

PCR products were ligated into pGEM-T easy (Promega) using standard protocols. Fragments encoding the genes of interest were removed using various restriction enzymes (Table 3) and ligated into restricted pET20b (Novagen). The resultant plasmid constructs were confirmed by sequencing. Correct constructs were transformed into *E. coli* BL21 using electroporation for subsequent expression analysis.

**Table 3: Cloning strategy for the generation of constructs expressing various nucleoside phosphorylases**

| Gene | Organism | Clone name | Restriction enzymes |
|---|---|---|---|
| *up* | *E. coli* | pETUP | *Nde*I, *Sal*I |
| BH1531 | *B. halodurans* | pMS-1531 | *Nde*I, *Hind*III |

### Expression of nucleoside phosphorylases

A single colony of *E. coli* BL21 expressing either BH1531 or UPase was inoculated into 5 ml LB broth containing 100 µg/ml ampicillin (final concentration).

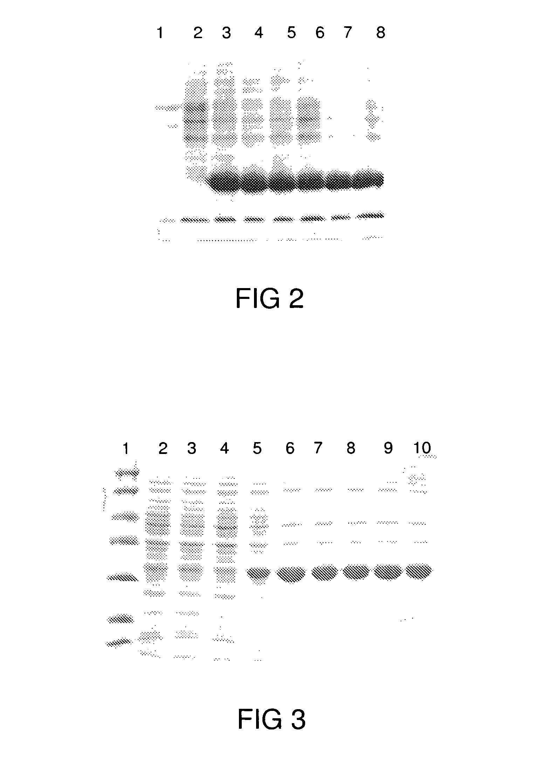

This culture was grown overnight at 37°C with shaking. One ml of the culture was diluted 1 in 100 into fresh LB broth. This fresh culture was grown at 37°C until the OD₆₀₀ reached 0.4. Expression of the nucleoside phosphorylases (Figures 2 and 3) was induced using 1 mM IPTG (final concentration) for between 3 to 14 hours.

### Production and preparation of nucleoside phosphorylases

Batch fermenters (Braun Biostat C) containing GMO 20 medium were inoculated with 800 ml inoculum. The composition of the GMO 20 medium was as follows: K₂HPO₄, 14.6 g/l; (NH₄)₂SO₄, 2 g/l; Na₂HPO₄, 3.6 g/l Citric Acid, 2.5 g/l; MgSO₄, 1.2 g/l; NH₄NO₃, 5 g/l and Yeast extract, 20 g/l.
Glucose 30.0 g/l and trace element solution, 5 ml/l were sterilized separately and added to the fermenters before inoculation. The trace element solution consists of the following: CaCl₂.2H₂O, 0.4 g/l; FeCl₃.6H₂O, 16.7 g/l; MnCl₂.4H₂O, 0.15 g/l; ZnSO₄.7H₂O, 0.18 g/l; CuCl₂.2H₂O. 0.125 g/l; CoCl₂.6H₂O, 0.18 g/l; Na₂EDTA, 20.1 g/l.
Ampicillin (100 mg/ml filter sterilised stock) was added to the medium to give a final concentration of 100 µg/ml).
Antifoam was aseptically added to the fermentation medium on demand.
The pH of the fermentations was maintained at pH 7.2 with 33% m/v NH₄OH and 20% m/v H₂SO₄.
The temperature was maintained at 37°C and the aeration set to 1 v/v/min. The initial agitation was set at 300 rpm and ramped up (automatic cascade) to control the pO₂ above 30% saturation. Growth, enzyme activity and glucose utilisation were followed by taking 10 ml samples at 1 hourly intervals. Enzyme expression was induced by addition of IPTG to a final concentration of 0.5 mM when the OD₆₆₀ reached a value of between 7 and 8 OD Units. The broth (20 I) was harvested 4 hours after induction and cooled to 4°C. The biomass was separated from the supernatant by continuous centrifugation (Beckman Avante, JCF-Z rotor, 14000 x g). The resultant pellet was resuspended in 2 litres 50 mM phosphate buffer, pH 8.0 and cells disrupted using a Constant Systems Cell Disruptor (2 passes at 20 kpsi). Cellular debris was removed by centrifugation (Beckman Avante, JA-10 rotor, 14000 x g). The resultant protein solution was lyophilised in the presence of 2% m/v maltose, 1% PEG 8000 (cryo-protectants). The lyophilisation procedure on a Vertis Genesis 25 I, was as follows: -35°C for 10 h; ramp to -5°C over 5 h; hold at -5°C for 5 h; ramp to 15°C over 20 h; hold at 15°C until dry.

### EXAMPLE 5

The use of *B. halodurans* PNPasc and *E. coli* PyNPase for the production of 5-methyluridine.

Guanosine (0.030 g, 0.106 mmol and 1.5% m/m), thymine (0.031 g, 0.246 mmol and 1.55% m/m) and sodium phosphate buffer (50 mM, 0.6% m/m, pH 7.5-8.0, ∼1.8 ml) were charged to a sample vial (∼ 5 ml). Stock solutions of PNPase and PyNPase enzymes were prepared using phosphate buffer and the required aliquots of PNPase (-30-200 µl) and PyNPase (-30-200 µl) added. The PNPase/PyNPase enzyme ratio was maintained at 1:1 at the following enzyme loading: PNPase, 0.27 U and PyNPase 0.27 U. The reaction mixture was stirred using a stirrer bar and heated to the required temperature (40°C). The reaction was typically carried out over 24 hours and then worked up, using sodium hydroxide solution (NaOH 10 M). The reaction mixture was then quantitatively analysed by HPLC for guanosine, guanine, thymine and 5-methyluridine. Typical results obtained show that a guanosine conversion > 90% and 5-methyluridine yield - 80% were achieved under the above conditions.

### EXAMPLE 6

The influence of pH on the production of 5-methyluridine by a combination of *B. halodurans* PNPase and *E. coli* PyNPase.

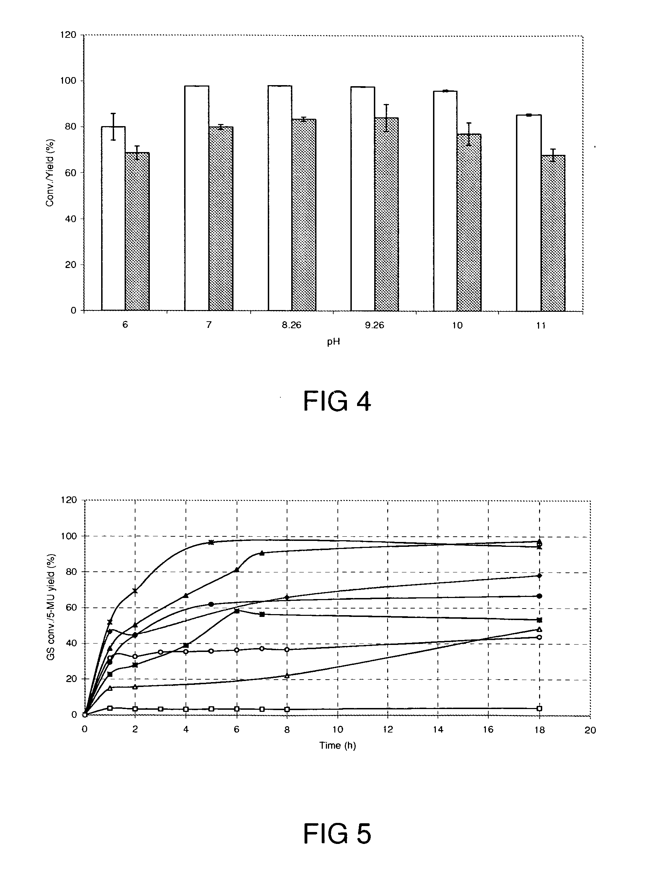

Experimental conditions similar to those described in Example 5 were used, except the pH of the reaction was altered to determine the operating range for this reaction. In order to obtain the desired pH, either 50 mM phosphate or carbonate buffer was used in combination with di-sodium hydrogen phosphate salt. Results are shown in Figure 4.

### EXAMPLE 7

The influence of temperature on the production of 5-methyluridine by a combination of *B. halodurans* PNPase and *E. coli* PyNPase.

Guanosine (15 g, 0.0530 mol and 1.5% m/m), thymine (15.4 g, 0.122 mol and 1.54% m/m) and sodium phosphate bluffer (50 mM, 0.6% m/m, pH 7.5-8.0, 969 g) were charged to a glass baffled reactor (2 I). Stock solutions of PNPase and PyNPase enzymes were prepared using phosphate buffer and the required aliquots of PNPase (10 - 30 ml) and PyNPase (10 - 30 ml) added. The PNPase/PyNPase enzyme ratio was maintained at 1:1 at the following enzyme loading: PNPase, 200 U and PyNPase 200 U. The reaction mixture was stirred using a stirrer bar and heated to the required temperature (40 - 70°C). The reaction was typically carried out over 24 hours and then worked up, using sodium hydroxide solution (NaOH 10 M). The reaction mixture was then quantitatively analysed by HPLC for guanosine, guanine, thymine and 5-methyluridine. Results are shown in Figure 5.

### EXAMPLE 8

Preparation of 5-methyluridine, using *B. halodurans* PNPase and *E. coli* PyNPase at 60°C at high substrate concentration.

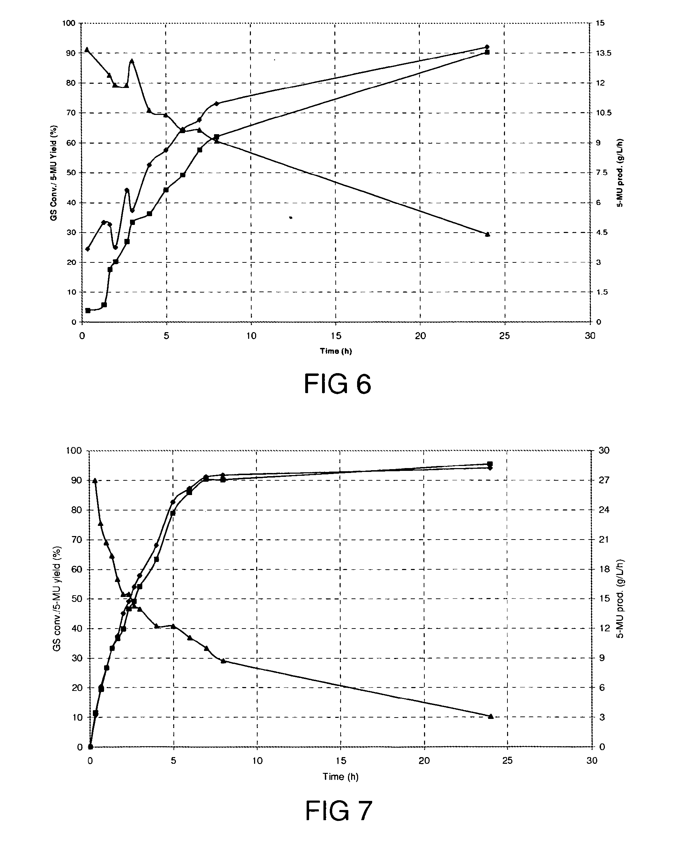

Guanosine (17.5 g 61.8 mmol, 12.9% m/m), thymine (17.4 g, 138 mmol, and 12.9% m/m) and phosphate buffer (101 g, 50 mM, pH 7.5 - 8) were charged to the reactor. The reaction mixture was heated to 60°C and stirred at 1000 rpm using a magnetic stirrer bar. The required amounts of PNPase (204 U) and, PyNPase (199 U) were then charged to the reactor. The reaction was conducted over 24 hours The 5-methyluridine productivity over the course of the reaction was between 4.5 -14 g/l/h. A guanosine conversion (> 90%), 5-methyluridine yield (∼90%) and 5-methyluridine productivity (-4.5 g/l/h) was obtained after 24 hours. Results are shown in Figure 6.

### EXAMPLE 9

### The optimal biocatalytic reaction

Phosphate buffer (1620 g, 50 mM, pH 7.5-8) was added to a suitable reactor, followed by guanosine solids (1040.09 g, 3.67 mole, 8.9% m/m) and then an additional 986 g phosphate buffer. Thymine (535.04 g, 4.2 mole, 4.8% m/m) was pre-wetted using 50 mM phosphate buffer (2000 g, pH 7.5-8) and then transferred to the reaction vessel. The balance of the phosphate buffer (5794 g) was then added to the reactor. The reaction mixture was stirred at 100 rpm using an anchor stirrer and heated to 60°C for 2 h prior to addition of the biocatalysts. During the heating procedure the required amount of PNPase (3.1119 g, 16006 U) and PyNPase (3.752 g, 16013 U) were pre-weighed and pre-dissolved in phosphate buffer, (-200 g, including rinse volumes). The reaction was conducted over 24 hours, however the reaction is typically complete within 8-12 hours at the required guanosine conversion (>90%) and 5-methyluridine yield (∼80-90%).

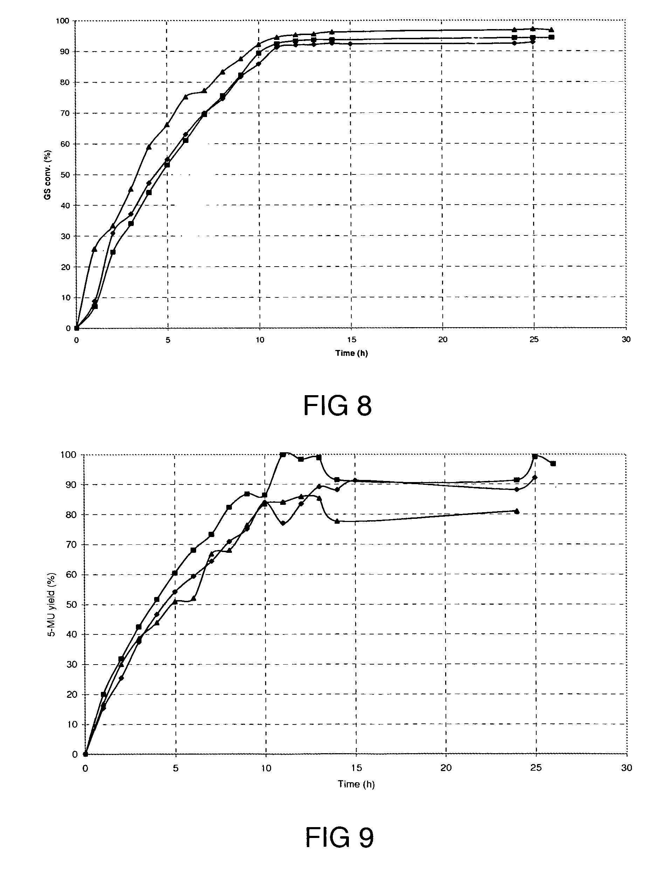

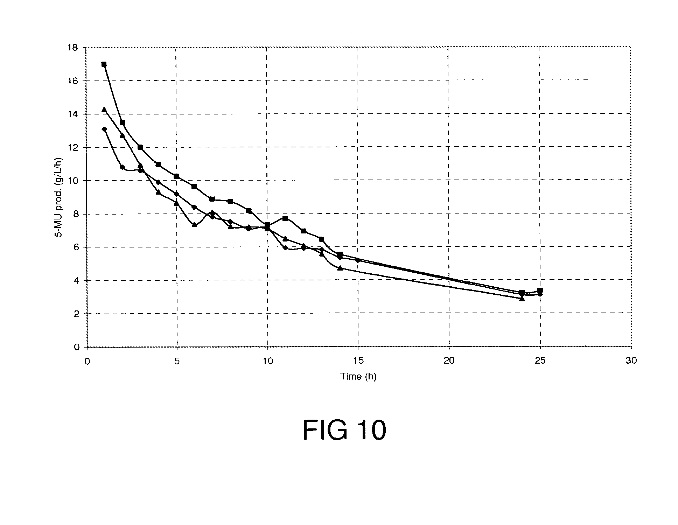

The optimal biocatalytic reaction was performed at scales ranging from 1 I (Figure 7) to 20 I (Figures 8 - 10). Replicate reactions to demonstrate the reproducibility and robustness of the 20 I reaction were performed and these results are shown in Figures 8-10.

### EXAMPLE 10

### The purification of 5-methyluridine

The reaction mixture was centrifuged hot (> 90°C) in order to recover guanine (∼90%) at a purity ∼90% m/m. Crude 5-methyluridine was then recovered by carrying out a water crystallization and water stripping process. The crude 5-methyluridine residues were then dried and further purified using a hot isobutanol filtration and crystallization process to remove inorganic salts (phosphate) and other organics such as thymine. The recovery of 5-methyluridine over the DSP process was ∼90% at a purity > 90% m/m.

The overall isolated 5-methyluridine yield was between 64 - 80%.

### EXAMPLE 11

### The synthesis of thymidine

### Preparation of 3',5'-Diacetyl-2'-bromothymidine (DABT)

To 5-methyluridine (10.0 g, 39 mmol) in a 3-necked round bottom flask fitted with a thermometer, reflux condenser and dropping funnel, was added acetonitrile (389 g). This slurry was heated to reflux and acetyl bromide (27.32 g, 222 mmol, 5.7 equivalents) was added dropwise over 30 min. During the course of addition, the solids dissolved to leave a yellow solution. The reaction was heated for an additional 30 min and then solvent was removed under reduced pressure through a NaOH solution trap and a solid NaOH trap. The residue was taken up in dichloromethane and this was washed twice with brine. The organic layer was dried over MgSO₄ (2 g), filtered and solvent removed to leave DAT as a beige residue (90% yield).

### Preparation of 3',5'-Diacetylthymidine (DAT)

DABT prepared above was dissolved in methanol sufficient to give a 20% reaction concentration. For example, 16.1 g solid from reaction above was dissolved in MeOH (64.4 g) and transferred to a Parr reactor. Sodium bicarbonate (4.6 g, 1.5 equivalents) and Ni catalyst A-5000 (4.5 g, 15% m/m catalyst loading, assume 50% slurry) were added. The H₂ pressure was set at 9 bar and the initial temperature was set to 35°C. The reaction is exothermic and cooling was employed in the first 30 min of reaction. After this time the reaction temperature was set to 40°C. After 3 h the reaction was stopped and filtered through celite (Celite 535 is preferred) to remove catalyst. Solvent was removed and the residue was taken up in ethyl acetate (90 ml). At this stage, a white solid precipitated from the ethyl acetate solution, which was removed by filtration. Solvent was removed *in vacuo* to leave DAT as a beige residue (90% yield).

### Preparation of Thymidine

To the DAT prepared above was added MeOH (37 ml) and NaOMe (1.4 g, 1.1 equivalents). The reaction was warmed at 40°C for 2 h. After this time additional MeOH was added (50 ml) and Amberlite resin (IR-120, 30 g) was added and stirred at room temperature. The beads were removed by filtration and the solvent was removed *in vacuo* to leave thymidine as a white solid (90% yield). Recrystallisation from MeOH gave rise to a product of high purity (>98%).

### EXAMPLE 12

### The synthesis of thymidine from 5-methyluridine obtained directly from the biocatalytic reaction after drying only, without further purification

### Preparation of 3',5'-Diacetyl-2'-bromothymidine (DABT)

To 5-methyluridine (12.5 g of 80% m/m purity, 39 mmol) in a 3-necked round bottomed flask fitted with a thermometer, reflux condenser and dropping funnel, was added acetonitrile (389 g). This slurry was heated to reflux and acetyl bromide (27.32 g, 222 mmol, 5.7 equivalents) was added dropwise over 30 min. During the course of addition, the solution became green in colour and remained slightly turbid. The reaction was heated for an additional 30 min and then solvent was removed under reduced pressure through a NaOH solution trap and a solid NaOH trap. The residue was taken up in dichloromethane and this was washed twice with brine. The solvent was removed to leave DAT as a dark brown residue (85% yield).

### Preparation of 3',5'-Diacetylthymidine (DAT)

DABT prepared above was dissolved in methanol sufficient to give a 20% reaction concentration and transferred to a Parr reactor. Sodium bicarbonate (4.6 g, 1.5 equivalents) and Ni catalyst A-5000 (4.6 g, 15% m/m catalyst loading, assume 50% slurry) were added. The H₂ pressure was set at 9 bar and the initial temperature was set to 35°C. The reaction is exothermic and cooling was employed in the first 30 min of reaction. After this time the reaction temperature was set to 40°C. After 3 h the reaction was stopped and filtered through celite (Celite 535 is preferred) to remove catalyst. Solvent was removed, the residue was taken up in ethyl acetate (90 ml) and MgSO₄ (2.5 g) was added. At this stage, a white solid precipitated from the ethyl acetate solution, which was removed by filtration. Solvent was removed *in vacuo* to leave DAT as a dark brown residue (71 % yield).

### Preparation of Thymidine

To the DAT prepared above was added MeOH (37 ml) and NaOMe (1.4 g, 1.1 equivalents). The reaction was warmed at 40°C for 2 h. After this time additional MeOH was added (50 ml) and Amberlite resin (IR-120, 30 g) was added and stirred at room temperature. The beads were removed by filtration and the solvent was removed *in vacuo* to leave thymidine as a white solid (91% yield). Recrystallisation from MeOH gave rise to a product of high purity, with a melting point of 187.8°C.

### EXAMPLE 13

Preparations of *B. halodurans* PNPase and *E. coli* PyNPase were immobilised (separately and co-immobilised) on polyethyleneimine backbone particles (ZA 2007/09300). In each case 200 µl of the enzyme preparation (100 µl of each preparation for co-immobilization) was added to 200 µl of backbone (5 mg/ml) and made up to 1.0 ml with deionised water. The solutions were incubated with mild agitation for 30 min. The particles were then washed 3 times with deionised water. The immobilised PNPase showed an activity of 0.26 U/mg particle (1 U was the amount of enzyme required to liberate 1 µmol uric acid per minute from inosine in the presence of xanthine oxidase) while the immobilised PyNPase showed an activity of 0.055 U/mg particle (1 U was the amount of enzyme required to liberate 1 µmol uracil from uridine in 1 minute). The co-immobilised preparation showed 1.13 and 1.49 U/mg of particle for PNPase and PyNPase respectively.

### Summary

Biocatalysts can provide a high reaction rate, high stereo-selectivity and regio-selectivity. There can also be a reduction in synthetic steps in synthesis and product isolation due to specificity (fewer by-products to separate out, protection steps eliminated). The multimeric enzymes PNPase and UPase/PyNPase are used simultaneously in this invention to synthesise nucleosides.

The use of low phosphate levels in the biocatalytic transglycosylation reaction of the invention allows for the shifting of the equilibrium of the reaction towards the formation of the products rather than the ribose phosphate intermediate.

The efficient transglycosylation of nucleosides is important for generation of thymidine and 5-methyluridine, which are, as indicated hereinbefore important precursors in the formation of the anti-retrovirals (ARV) AZT and stavudine.

Purine nucleoside phosphorylase (PNPase) is used to remove the ribose-1-phosphate moiety from the guanosine nucleoside. The ribose-1-phosphate is then transferred to thymine by pyrimidine nucleoside phosphorylase (PyNPase) to generate 5-methyluridine. Hence a two enzyme biocatalyst system is, in accordance with the invention, used to achieve nucleoside transformation effectively. In accordance with the invention, the purified enzymes are used together to synthesise 5-methyluridine from the cheap starting material, guanosine. The process used involves the enzymatic cleavage of guanosine into the insoluble guanine, and the highly soluble ribose-1-phosphate by PNPase. The ribose-1-phosphate is then covalently bound to thymine by the UPase to give 5-methyluridine. Results show a greater than 80% yield, and typically around 80-90% yield of 5-methyluridine from guanosine.

5-Methyluridine is a relatively expensive intermediate in the formation of AZT or stavudine. By decreasing the cost of this intermediate, it is feasible to decrease the cost of several anti-retroviral drugs. The use of biocatalysts allows for the highly specific catalysis with low by-product formation. The use of the selected *B. halodurans* enzymes allows for improved yields and reaction kinetics.

The invention thus provides a high yield and rapid chemo-enzymatic process for the preparation of β-thymidine from guanosine and thymine. Preparation of the intermediate 5-methyluridine is achieved in good yield by biocatalytically converting guanosine and thymine into 5-methyluridine and guanine using the enzymes purine nucleoside phosphorylase and pyrimidine nucleoside phosphorylase. The catalytic enzymes are produced by over-expression of an *E. coli* pyrimidine nucleoside phosphorylase and a *B. halodurans* purine nucleoside phosphorylase in *E. coli* hosts. High yield production of both biocatalysts has been demonstrated in batch fermentation of the respective strains. The combination of these two enzymes yielded 80-90% 5-methyluridine from guanosine with typical productivities of 7-11 g/l/h at 60°C. Subsequent chemical conversions of 5-methyluridine gave rise to high-purity β-thymidine in three steps.

### References

Esipov, R. S., Gurevich, A. I., Chuvikovsky, D. V., Chupova, L. A., Muravyova, T. 1. and Miroshnikov, A. I. (2002) Overexpression of Escherichia coli Genes Encoding Nucleoside Phosphorylases in the pET/BI21(DE3) System Yields Active Recombinant Enzymes. Protein Expr Purif. 24, 56-60.

**SEQUENCE LISTING**

Sequence ID No. 1 : BH1531 amino acid sequence

MLNVTQLQEATTFIQQQIETKPTIGLILGSGLGILADEIEQPVKVPYSDIPHFPVST

VQGHAGQLVIGMLEGKQVIAMQGRFHFYEGYSLEVVTFPVRVMKALGVEQIIVTNAA

GGVNESFEAGDLMIIRDHINNMAQNPLIGPNDEAFGVRFPDMSNAYSERLRTLAKEK

GNTLNLKLQEGVYVANTGPVYETPAEVRMIRKLGGDAVGMSTVPEVIVARHAGLEVL

GISCISNMAAGILPQPLSHDEVIETTERVRQDFLNLVKAIVKDM#

Sequence ID No. 2 : E.coli UPase amino acid sequence

MSKSDVFHLGLTKNDLQGATLAIVPGDPDRVEKIAALMDKPVKLASHREFTTWRAEL

DGKPVIVCSTGIGGPSTSIAVEELAQLGIRTFLRIGTTGAIQPHINVGDVLVTTASV

RLDGASLHFAPLEFPAVADFECTTALVEAAKSIGATTHVGVTASSDTFYPGQERYDT

YSGRVVRHFKGSMEEWQAMGVMNYEMESATLLTMCASQGLRAGMVAGVIVNRTQQEI

PNAETMKQTESHAVKIVVEAARRLL#

Sequence ID No. 3: BH1533 amino acid sequence

MRMVDLIEKKRDGLALTKEEIDFIISGYTNGDIPDYQMSAFAMAIFFQDMTADERAY

LTMAMAESGDQIDLSGIEGVKVDKHSTGGVGDKTTIALAPLVASVGVPVAKMSGRGL

GHTGGTIDKLESIAGFNVEIPTERFLELVNKNKLAVVGQTANLTPADKKLYGLRDVT

ATVNSIPLIASSIMSKKIASGANAIVLDVKTGSGAFMKELEQSKELAEAMVEIGNNL

GRQTMAVISDMNQPLGWAVGNALEVKEAIDMLRGEGPDDLRELCLTLGSHMVFLAKQ

ATSVADARQKLEEAISSGKALDTLKTFISAQNGDPSVVDNPQLLPTAQFQIEVKAEE

NGHVASIVADRIGTAAMVLGAGRATKESDIDLSVGIVLKKKMGESVQAGETLAILHA

NKEDVQEVIDRVIKAYHISPDPVAKPTLIYEEIH#

Sequence ID No. 4 : BH1531 nucleotide sequence

ATGCTTAACGTAACTCAATTGCAAGAAGCGACTACATTTATTCAACAGCAAATAGAA
ACAAAACCAACGATCGGTTTAATTTAGGTTCTGGTTTAGGGATTTTAGCTGATGAG
ATCGAACAGCCGGTGAAAGTTCCTTACAGTGACATTCCACATTTTCCTGTCTCTACC
GTCCAAGGACATGCGGGCCAGCTTGTGATCGGCATGCTTGAAGGAAAGCAAGTGATT
GCGATGCAAGGGCGATTTCATTTTTATGAAGGCTACAGCCTTGAGGTTGTCACATTC
CCTGTCCGCGTCATGAAAGCTCTAGGTGTAGAACAAATCATTGTCACAAATGCAGCG
GGCGGTGTGAACGAATCGTTCGAAGCGGGTGACCTAATGATCATTCGCGACCATATT
AACAACATGGCACAAAACCCACTAATTGGGCCGAATGATGAGGCGTTCGGCGTACGT
TTTCCGGATATGTCAAATGCCTACTCTGAGCGCTTACGAACGTTAGCGAAGGAAAAG
GGAAACACGTTGAATCTCAAGCTACAGGAAGGGGTCTATGTTGCGAACACAGGTCCT
GTTTATGAAACCCCTGCTGAAGTGCGGATGATTCGAAAACTTGGCGGAGATGCTGTA
GGTATGTCAACCGTACCAGAAGTCATTGTCGCTCGTCATGCAGGACTTGAGGTTCTT
GGAATTTCATGTATTTCTAACATGGCGGCAGGAATTTTACCACAGCCTTTATCACAT
GATGAAGTCATTGAAACAACGGAACGAGTGAGGCAAGACTTTCTTAATCTTGTCAAA
GCAATCGTTAAAGACATGTAA

Sequence ID No 5 : E. coli UPase nucleotide sequence

ATGTCCAAGTCTGATGTTTTTCATCTCGGCCTCACTAAAAACGATTTACAAGGGGCT
ACGCTTGCCATCGTCCCTGGCGACCCGGATCGTGTGGAAAAGATCGCCGCGCTGATG
GATAAGCCGGTTAAGCTGGCATCTCACCGCGAATTCACTACCTGGCGTGCAGAGCTG
GATGGTAAACCTGTTATCGTCTGCTCTACCGGTATCGGCGGCCCGTCTACCTCTATT
GCTGTTGAAGAGCTGGCACAGCTGGGCATTCGCACCTTCCTGCGTATCGGTACAACG
GGCGCTATTCAGCCGCATATTAATGTGGGTGATGTCCTGGTTACCACGGCGTCTGTC
CGTCTGGATGGCGCGAGCCTGCACTTCGCACCGCTGGAATTCCCGGCTGTCGCTGAT
TTCGAATGTACGACTGCGCTGGTTGAAGCTGCGAAATCCATTGGCGCGACAACTCAC
GTTGGCGTGACAGCTTCTTCTGATACCTTCTACCCAGGTCAGGAACGTTACGATACT
TACTCTGGTCGCGTAGTTCGTCACTTTAAAGGTTCTATGGAAGAGTGGCAGGCGATG
GGCGTAATGAACTATGAAATGGAATCTGCAACCCTGCTGACCATGTGTGCAAGTCAG
GGCCTGCGTGCCGGTATGGTAGCGGGTGTTATCGTTAACCGCACCCAGCAAGAGATC
CCGAATGCTGAGACGATGAAACAAACCGAAAGCCATGCGGTGAAAATCGTGGTGGAA
GCGGCGCGTCGTCTGCTGTAA

Sequence ID No. 6 : BH1533 nucleotide sequence

ATGCGCATGGTTGACTTAATCGAAAAAAGCGTGACGGTCTCGCATTAACGAAAGAA
GAGATTGATTTATCATTTCAGGCTATACGAACGGTGATATTCCCGACTATCAAATG
TCGGCGTTTGCGATGGCGATTTTCTTTCAAGATATGACAGCAGATGAACGAGCGTAT
TTGACGATGGCAATGGCTGAATCAGGCGATCAAATCGACCTCTCTGGCATTGAAGGT
GTTAAGGTTGATAAACATTCCACAGGAGGCGTTGGCGATAAGACGACGATTGCCCTT
GCTCCCCTCGTGGCATCGGTTGGTGTTCCTGTTGCCAAAATGTCTGGAAGGGGGCTC
GGGCATACAGGTGGAACGATTGACAAGCTAGAGTCGATCGCAGGTTTTAACGTGGAA
ATTCCGACGGAGCGCTTTCTTGAGCTTGTGAACAAAAATAAGCTTGCCGTCGTTGGG
CAAACAGCGAATTTAACCCCTGCCGATAAAAAGTTGTACGGCCTTCGCGATGTGACC
GCAACGGTGAATTCGATTCCGCTCATTGCCAGCTCGATCATGAGCAAAAAAATAGCT
TCAGGCGCTAACGCCATCGTTCTCGATGTAAAAACAGGGTCAGGGGCGTTTATGAAA
GAGCTTGAGCAATCAAAAGAGCTTGCCGAAGCGATGGTGGAAATCGGCAACAACCTT
GGAAGGCAAACAATGGCGGTTATTTCTGATATGAATCAGCCGCTTGGATGGGCTGTT
GGAAATGCCCTTGAAGTGAAGGAAGCAATTGATATGCTTCGGGGCGAGGGACCAGAT
GATTTGCGCGAGCTTTGTTTAACCCTTGGTAGCCATATGGTCTTTTTAGCGAAACAA
GCTACTTCCGTGGCAGACGCCCGTCAAAAGCTAGAGGAAGCGATTTCCTCTGGTAAA
GCACTGGACACACTAAAAAACGTTCATTAGTGCGCAAAACGGTGATCCATCTGTAGTA
GACAATCCGCAGCTTCTTCCAACTGCCCAATTCCAAATAGAAGTAAAGGCAGAGGAA
AATGGACATGTAGCGTCGATTGTTGCTGACCGTATTGGTACTGCAGCGATGGTTCTC
GGAGCTGGACGAGCAACGAAGGAATCGGACATTGATTTGTCTGTTGGAATCGTTCTG
AAGAAGAAAATGGGTGAGTCGGTGCAAGCAGGTGAAACGCTTGCGATTCTTCATGCA
AACAAAGAGGATGTCCAAGAAGTGATCGATAGGGTGATTAAAGCCTATCACATCTCT
CCTGATCCTGTCGCAAAGCCAACGCTCATTTACGAGGAGATTCATTAA

## Claims

1. A process for converting guanosine to 5-methyluridine by means of biocatalytic transglycosylation, with the biocatalyst comprising a combination of a purine nucleoside phosphorylase from *Bacillus halodurans,* PNPase (BH1531) of sequence ID No 1, or a protein with at least 90% sequence similarity thereto, and a pyrimidine nucleoside phosphorylase (PyNPase).

2. The process of Claim 1, wherein the pyrimidine nucleoside phosphorylase is from *Escherichia coli,* being UPase of sequence ID No 2, or a protein with at least 90% sequence similarity thereto.

3. The process of Claim 1, wherein the pyrimidine nucleoside phosphorylase is from *Bacillus halodurans,* being PyNPase (BH1533) of sequence ID No 3, or a protein with at least 90% sequence similarity thereto.

4. The process of Claim 2, wherein the purine nucleoside phosphorylase and pyrimidine nucleoside phosphorylase are those produced by batch fermentation of *Escherichia coli* JM109 (DE3) [pMS1531] and *Escherichia coli* BL21 (DE3) [pETUP] respectively.

5. The process of any one of Claims 1 to 4 inclusive, wherein the transglycosylation is performed at a temperature between 30°C and 70°C.

6. The process of any one of Claims 1 to 5 inclusive, wherein the transglycosylation is effected at a pH in the range of 6 to 11.

7. The process of any one of Claims 1 to 6 inclusive, wherein the molar yield of 5-methyluridine is 80% to 90%.

8. The process of any one of Claims 1 to 7 inclusive, wherein the transglycosylation reaction productivity is between 0.5 and 27 g/l/h.

9. The method of any one of Claims 1 to 8 inclusive, wherein the biocatalyst is immobilized on a backbone.

10. The process of any one of Claims 1 to 9 inclusive, which includes chemically manipulating the 5-methyluridine, to obtain therefrom ß-thymidine.

11. The process of Claim 10, wherein the chemical manipulation of the 5-methyluridine is effected without any purification thereof after it has been obtained by the conversion of the guanosine.

12. A process according to Claim 10 or Claim 11, wherein the chemical manipulation of the 5-methyluridine includes reacting the 5-methyluridine with hydrogen bromide or acetyl bromide in acetonitrile.

13. A biocatalyst which comprises the combination of a pyrimidine nucleoside phosphorylase (PyNPase) and a purine nucleoside phosphorylase from *Bacillus halodurans,* PNPase (BH1531) of sequence ID No 1, or a protein with at least 90% sequence similarity thereto.

## Patentansprüche

1. Verfahren zum Umwandeln von Guanosin zu 5-Methyluridin mittels biokatalytischer Transglycosylierung mit eine Biokatalysator, welcher eine Mischung aus einer Purin-Nukleosid-Phosphorylase aus *Bacillus halodurans*, PNPase (BH1531) mit der Sequenz ID No 1 oder einem Protein mit wenigstens 90% Sequenzähnlichkeit hierzu sowie einer Pyrimidin-Nukleosid-Phosphorylase (PyNPase) enthält.

2. Verfahren nach Anspruch 1, wobei die Pyrimidin-Nukleosid-Phosphorylase aus *Escherichia coli* stammt und eine UPase mit der Sequenz ID No 2 oder ein Protein mit weinigstens 90% Sequenzähnlichkeit hierzu ist.

3. Verfahren nach Anspruch 1, wobei die Pyrimidin-Nukleosid-Phosphorylase aus *Bacillus halodurans* stammt und PyNPase (BH1533) mit der Sequenz ID No 3 oder ein Protein mit wenigstens 90% Sequenzähnlichkeit hierzu ist.

4. Verfahren nach Anspruch 2, wobei die Purin-Nukleosid-Phosphorylase und die Pyrimidin-Nukleosid-Phosphorylase solche sind, welche durch Chargenfermentation von *Escherichia coli* JM109 (DE3) [pMS1531] bzw. *Escherichia coli* BL21 (DE3) [pETUP] hergestellt worden sind.

5. Verfahren nach einem der Ansprüche 1 bis 4, wobei die Transglycosylierung bei einer Temperatur zwischen 30°C und 70°C durchgeführt wird.

6. Verfahren nach eine der Ansprüche 1 bis 5, wobei die Transglycosylierung bei eine pH-Wert in einem Bereich zwischen 6 und 11 durchgeführt wird.

7. Verfahren nach einem der Ansprüche 1 bis 6, wobei die molare Ausbeute an 5-Methyluridin 80% bis 90% beträgt.

8. Verfahren nach eine der Ansprüche 1 bis 7, wobei die Produktivität der Transglycosylierung zwischen 0,5 und 27 g/l/Std. beträgt.

9. Verfahren nach eine der Ansprüche 1 bis 8, wobei der Biokatalysator an einen Rückgrat immobilisiert ist.

10. Verfahren nach einem der Ansprüche 1 bis 9, welches das chemische Manipulieren von 5-Methyluridin umfasst, um daraus β-Thymidin zu erhalten.

11. Verfahren nach Anspruch 10, wobei das chemische Manipulieren des 5- Methyluridins ohne eine Reinigung hiervon durchgeführt wird, nachdem dieses durch Umwandlung des Guanosins erhalten worden ist.

12. Verfahren nach Anspruch 10 oder 11, wobei das chemische Manipulieren des 5- Methyluridins das Reagieren des 5- Methyluridins mit Bromwasserstoff oder mit Acetylbromid in Acetonitril umfasst.

13. Biokatalysator, welcher eine Mischung aus einer Pyrimidin-Nukleosid-Phosphorylase (PyNPase), einer Purin-Nukleosid-Phosphorylase aus *Bacillus halodurans* sowie PyNPase (BH1531) mit der Sequenz ID No 1 oder einem Protein mit wenigstens 90% Sequenzähnlichkeit hierzu enthält.

## Revendications

1. Processus pour convertir la guanosine en 5-méthyluridine par le biais d'une transglycosylation biocatalytique, le biocatalyseur comprenant une combinaison de purine nucléoside phosphorylases issue de la protéine *Bacillus halodurans*, PNPase (BH1531) de séquence SEQ ID No. 1, ou d'une protéine avec une séquence qui lui est similaire à au moins 90%, et de pyrimidine nucléoside phosphorylase (PyNPase).

2. Processus de la revendication 1, dans lequel la pyrimidine nucléoside phosphorylase est issue de la protéine *Escherichia coli*, étant la UPase de séquence SEQ ID No. 2, ou d'une protéine avec une séquence qui lui est similaire à au moins 90%.

3. Processus de la revendication 1, dans lequel la pyrimidine nucléoside phosphorylase issue de la protéine *Bacillus halodurans*, étant la PyNPase (BH1533) de séquence SEQ ID No. 3, ou d'une protéine avec une séquence qui lui est similaire à au moins 90%.

4. Processus de la revendication 2, dans lequel la purine nucléoside phosphorylase et la pyrimidine nucléoside phosphorylase sont produites par fermentation classique de la protéine *Escherichia coli* JM109 (DE3) [pMS1531] et *Escherichia coli* BL21 (DE3) [pETUP] respectivement.

5. Processus de l'une quelconque des revendications 1 à 4 incluses, dans lequel la transglycosylation est conduite à une température comprisse entre 30°C et 70 °C.

6. Processus de l'une quelconque des revendications 1 à 5 incluses, dans lequel la transglycosylation est conduite à un pH dans la plage de 6 à 11.

7. Processus de l'une quelconque des revendications 1 à 6 incluses, dans lequel le rendement molaire de la 5-méthyluridine est de 80% à 90%.

8. Processus de l'une quelconque des revendications 1 à 7 incluses, dans lequel la productivité de la réaction de transglycosylation est comprise entre 0,5 et 27 g/l/h.

9. Procécé de l'une quelconque des revendications 1 à 8 incluses, dans lequel le biocatalyseur est immobilisé sur un squelette.

10. Processus de l'une quelconque des revendications 1 à 9 incluses, qui comporte la manipulation chimique de la 5-méthyluridine, afin d'en obtenir la β-thymidine.

11. Processus de la revendication 10, dans lequel la manipulation chimique de la 5-methyluridine est réalisée sans purification aucune de celle-ci après qu'elle a été obtenue par la conversion de la guanosine.

12. Processus selon la revendication 10 ou 11, dans lequel la manipulation chimique de la 5-methyluridine comporte la réaction de la 5-methyluridine avec le bromure d'hydrogène ou le bromure d'acétyle dans l'acétonitrile.

13. Biocatalyseur qui comprend la combinaison de pyrimidine nucléoside phosphorylase (PyNPase) et de la purine nucléoside phosphorylase à partir de la protéine *Bacillus halodurans,* PyNPase (BH1531) de séquence SEQ ID No. 1, ou d'une protéine avec une séquence qui lui est similaire à au moins 90%.
